# EUROPEAN PATENT APPLICATION

(11) **EP 2 591 814 A1**
(43) Date of publication of application: **15.05.2013**
(21) Application number: 11188823.6
(22) Date of filing: 11.11.2011
(51) Int. Cl.: A61M 5/00

(54) **Needle storage magazine**

(71) Applicant: Sanofi-Aventis Deutschland GmbH, 65929 Frankfurt (DE)
(72) Inventor: The designation of the inventor has not yet been filed

(57) **Abstract**

It is described a needle storage magazine (1, 101 to 401) for storing and dispensing a plurality of needle assemblies (2, 102 to 402, 2', 202' to 402'), the needle storage magazine (1, 101 to 401) comprising:
- a plurality of mounting sleeves (1.2, 101.2 to 401.2) each adapted to mount an unused needle assembly (2, 102 to 402) or a used needle assembly (2', 102' to 402'),
- a case (1.1, 101.1 to 401.1) holding the mounting sleeves (1.2, 101.2 to 401.2) side by side, and
- a feeding and storage mechanism (1.3, 101.3 to 401.3) which feeds one of the unused needle assemblies (2, 102 to 402) within the mounting sleeve (1.2, 101.2 to 401.2) to an opening (1.4, 101.4 to 401.4) as well as which draws back one of the used needle assemblies (2', 102' to 402') into the mounting sleeve (1.2, 101.2 to 401.2).

## Description

### Technical Field

The present invention relates to a needle storage magazine for medical needles.

### Background of the Invention

Medical needles and/or needle assemblies are often stored in a sterile compartment.

### Summary of the Invention

It is an object of the present invention to provide an improved needle storage magazine for medical needles.

The object is achieved by a needle storage magazine according to claim 1.

Preferred embodiments of the invention are given in the dependent claims.

According to the invention a needle storage magazine for storing and dispensing a plurality of needle assemblies comprises:
- a plurality of mounting sleeves each adapted to mount an unused needle assembly or a used needle assembly,
- a case holding the mounting sleeves side by side, and
- a feeding and storage mechanism which feeds one of the unused needle assemblies within the mounting sleeve to an opening as well as which draws back one of the used needle assemblies into the mounting sleeve.

The invention provides easy storage and transport of an amount of needles in addition to an easy and safe handling to take out unused needles from the needle storage magazine and put used needles back to the magazine.

The use of single needles has the advantage that a considerably higher packing density can be achieved than by the use of common needles, e.g. insulin needles. Thus, the user can take along comfortably a needle ration for various days or weeks.

In an exemplary embodiment, the feeding and storage mechanism is designed as a gear drive. A gear drive provides low operating forces, easy handling and comfortable design.

In a further exemplary embodiment, the feeding and storage mechanism comprises at least two gear rods and between them at least one gear wheel is movably arranged. In particular, the gear drive comprises two gear rods with integrated gear rims which interact with teeth of respective two gear wheels. This allows a simple way of feeding and storing needles out of or into the magazine.

In another exemplary embodiment, the mounting sleeve comprises at its end directed to the feeding and storage mechanism a latching element and one of the gear rod comprises at its end directed to the opening a corresponding latching element. The corresponding latching elements allow an easy releasable snap-fit connection.

In an exemplary embodiment, the opening is formed into a case wall, whereby the opening is coverable by a cover element comprising a cavity and which is movable with respect to the case. When not using the magazine, the opening can be fully closed by the cover element. Furthermore, the opening can be moved relative to the case in such a manner that the cavity is positioned above a full mounting sleeve for removing an unused needle assembly or above an empty mounting sleeve for storing a used needle assembly into the mounting sleeve.

In an exemplary embodiment, the mounting sleeves are radially arranged and form together a full circle. The radial arrangement provides a high packing density and a handy shape as well as an easy pocket transport of the magazine.

In an exemplary embodiment, the mounting sleeves are rotatably arranged within the case in at least one direction and are locked into the opposite direction. Locking the rotation of the mounting sleeves into one of the direction prevents an unintentional reuse of used and stored needles. Furthermore, it provides a safe disposal of used needles and prevents injections and injuries.

In a further exemplary embodiment, the case comprises a closing mechanism closing a retaining mechanism of the mounting sleeve for irreversible retaining an used needle assembly within the mounting sleeve.

In an exemplary embodiment, the retaining mechanism comprises at least one retaining clip radially arranged on an inner surface of the mounting sleeve on the end of the mounting sleeve directed to the opening.

According to a further exemplary embodiment, the retaining clip is closed by the closing mechanism when a cavity of a cover element is moved from a feeding position to a disposal position.

In another exemplary embodiment, each mounting sleeve comprises a needle support arranged within the mounting sleeve. In an exemplary embodiment, the needle support has a profile shape to support the needle assembly in an upright position.

In an exemplary embodiment, the needle support has a L-profile to provide a latching element by a bent arm directed to a gear drive.

In an alternative exemplary embodiment, the needle support has a U-profile to support the needle assembly in an upright position whereby the end of the U-profile directed to a gear drive comprises a bent latching element.

In an exemplary embodiment, the needle assembly comprises a friction ring. The needle is held in the mounting sleeve without a needle support only by friction between a cone on the needle and an elastomer ring. Due to the reduced and compact mounting assembly of the needle in the mounting sleeve the mounting sleeve diameter may be reduced to fit more needles in the magazine.

In a further exemplary embodiment, the case comprises at least one surface that protrudes inwards to bend used needle. This provides another possible solution, instead of pulling the used needle back into the mounting sleeve, the needle ends brush along a contour that bends them so that they do not hinder the pen to enter the neighbouring cavity for storing the next used needle.

In another exemplary embodiment, each needle assembly comprises a needle mounted on a needle hub wherein the needle hub comprises a protruding element on the end which is directed inwards the mounting sleeve to be irreversibly lockable at retaining clips.

In a further exemplary embodiment, the opening is coverable by a cover element.

In an exemplary embodiment, the cover element comprises an outer gripping surface and a cavity.

The needle storage magazine according to the invention will be used for save and easy storage and transport of an amount of needles for injection pens (e.g. for dispensing insulin). The needle storage magazine will be connected to the insulin pen so that the needles can be mounted and demounted automatically to or from the insulin vial. This augments the safety and comfort of the apparatus.

The use of single needles has the advantage that a considerably higher packing density can be achieved than by using common insulin needles. So the patient can take along comfortably a needle ration for a complete insulin vial.

As the needles are integrated into the dispensing device the user does not need to carry along several objects. This augments the comfort and also the safety as the user cannot forget to take along a sufficient number of injection needles.

The automatic needle mounting and demounting system augments the safety of the insulin injection. It is no more possible that the user damages the needle when mounting it to the pen, which can cause malfunction. Additionally the user is protected from needle-stick injuries when mounting or demounting the needle. Needle-stick injuries can transfer diseases such as Hepatitis or HIV, for example from patients to healthcare professionals.

The automatic mounting and demounting system can augment the patient's comfort considerably as the insulin injection process is easier and faster. This is important because insulin patients need to have injections several times a day and therefore injections also have to be performed in public places, such as restaurants. The system prevents the user from embarrassing situations.

By automatically removing the sterile blister the user does no longer need to remove the sterile barrier manually. This simplifies the use and does not produce any waste. The integrated reuse prevention protects the user from unintentional reuse of a needle. It also provides a safe disposal of used needles and prevents infections and injuries because of neglectfully stored needles.

As the used needles are stored inside the case of the needle storage magazine, there is no waste during the use of the needle storage magazine. If the needle storage magazine is disposed the used needles are protected, so there is no injury risk for people who handle the waste.

The needle storage magazine according to the invention allows for making insulin injections without exposing the needle to the patient, which is beneficial for patients who suffer from belonephobia.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

### Brief Description of the Drawings

The present invention will become more fully understood from the detailed description given hereinbelow and the accompanying drawings which are given by way of illustration only, and thus, are not limitive of the present invention, and wherein:
- Figure 1: shows in a perspective view an exemplary embodiment of a needle storage magazine with a non-transparent case in a starting or rest position.
- Figure 2: shows in a perspective view an exemplary embodiment of a needle storage magazine with a partly transparent case in a feeding or reinsertion position.
- Figure 3: shows in a perspective view an exemplary embodiment of a needle storage magazine with a transparent case in a storage or disposal position.
- Figure 4: shows in a sectional view an exemplary embodiment of a mounting sleeve.
- Figure 5: shows in a sectional view another exemplary embodiment of a mounting sleeve.
- Figure 6: shows an enlarged section of an exemplary embodiment of a needle storage magazine with an uncovered opening aligned with a cavity.
- Figure 7: shows four enlarged sections of an exemplary embodiment of a needle storage magazine with a cover cavity in different positions.
- Figures 8 to 10: show sectional views of an exemplary embodiment of a feeding and storage mechanism in different positions.
- Figure 11: shows in a sectional view an exemplary embodiment of a needle storage magazine with used needle assemblies mountable/mounted in mounting sleeves in different positions.
- Figure 12: shows in an enlarged section an exemplary embodiment of a mounting sleeve connectable to a feeding and storage mechanism.

- Figure 13: shows in a top view an exemplary embodiment of mounting sleeves with mounted needle assemblies in different positions.
- Figure 14: shows in a sectional view an exemplary embodiment of a needle storage magazine with different used needle assembly mountable/mounted in a corresponding mounting sleeve.
- Figure 15: shows in sectional view an exemplary embodiment of a mounting sleeve.
- Figure 16: shows in a sequence of sectional views and corresponding top views an exemplary embodiment of a needle storage magazine with used needle assemblies mountable/mounted in mounting sleeves in different positions.
- Figure 17: shows in another sequence of sectional views an exemplary embodiment of a needle storage magazine with used needle assemblies mountable/mounted in mounting sleeves in different positions.
- Figures 18 to 19: show in top views an exemplary embodiment of a needle storage magazine with an uncovered and partly covered opening.
- Figure 20: shows a perspective view of an exemplary embodiment of a needle storage magazine.

Corresponding parts are marked with the same reference symbols in all figures.

### Detailed Description of Preferred Embodiments

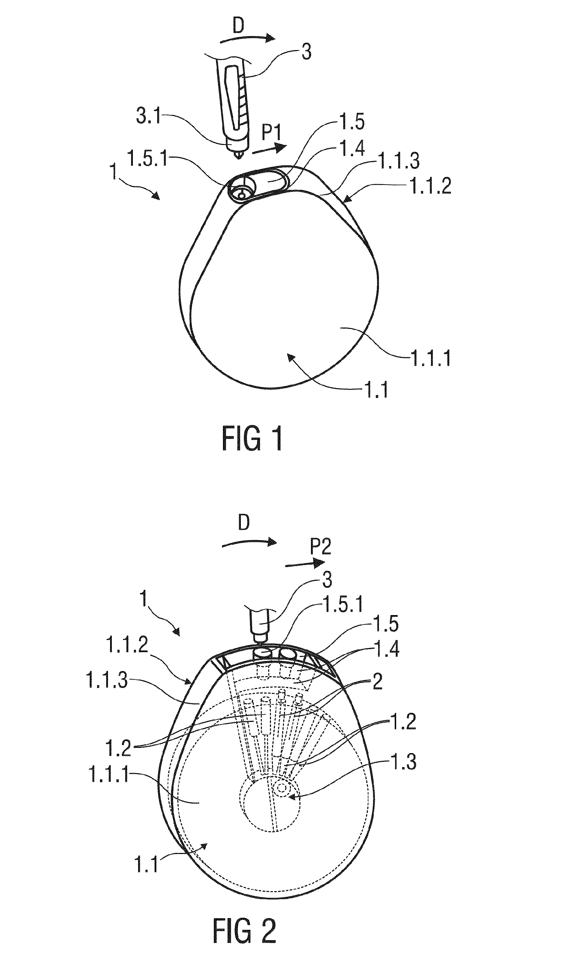

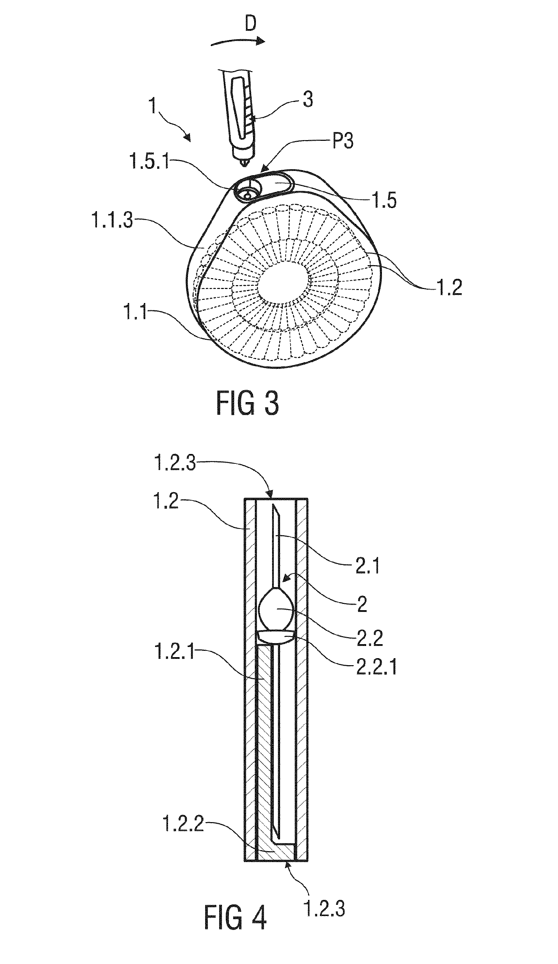

Figures 1 to 3 show in perspective views exemplary embodiments of a needle storage magazine 1 according to the invention in different positions: figure 1 in a starting or rest position, figure 2 in a feeding (dispensing/withdrawal) or reinsertion position and figure 3 in a storage or disposal position.

The needle storage magazine 1 is adapted to dispense and store a number of unused sterile needle assemblies 2 as well as to finally store used needle assemblies 2' so that a new sterile needle assembly 2 can be delivered for each new injection and a used needle assembly 2' can be stored back into the needle storage magazine 1 after using, e.g. after injection.

The needle storage magazine 1 comprises a case 1.1 for holding a number of mounting sleeves 1.2. The needle storage magazine 1 has a substantially cylindrical shape with outer surfaces 1.1.1 and 1.1.2 and a circumferential edge 1.1.3. Alternatively, the mounting sleeves 1.2 may be designed as cuboids or tubes with an elongate shape.

The circumferential edge 1.1.3 comprises at least one opening 1.4. The opening 1.4 is formed into the circumferential edge 1.1.3 and has a size which corresponds to the outer dimensions of two mounting sleeves 1.2 next to each other. Over the opening 1.4 a movable cover element 1.5 is arranged.

The cover element 1.5 is substantially rounded and comprises a cavity 1.5.1. The cover element 1.5 is movable with respect to the case 1.1 by a sliding assembly 1.6 according to arrows P1 to P3. The cavity 1.5.1 has approximate dimensions which correspond to outer dimensions of an injection device 3, e.g. of an insulin pen.

Figure 1 shows the cavity 1.5.1 of the cover element 1.5 in a rest position besides the opening 1.4 of the case 1.1 where an access to the opening 1.4 and thus to the stored unused and used needle assemblies 2, 2' is covered by the edge 1.1.3 of the case 1.1 and an injection device 3 cannot be inserted. Figure 2 shows the cavity 1.5.1 aligned to one side of the opening 1.4 of the case 1.1 in a feeding or reinsertion position in which an unused needle assembly 2 can be taken out by an injection device 3 or a used needle assembly 2' can be reinserted into the needle storage magazine 1. The disposal position of the cavity 1.5.1, in which the cavity 1.5.1 is aligned to the other side of the opening 1.4, is not shown. Figure 3 shows the cavity 1.5.1 back to the rest position after storing of the used needle assembly 2'.

As it is further shown in figure 3 by a transparent case 1.1 the mounting sleeves 1.2 are arranged within the case 1.1 side by side. Especially, the mounting sleeves 1.2 are radially arranged and form together a full circle. Furthermore, the mounting sleeves 1.2 are rotatably arranged within the case 1.1 in at least one direction D. Into the opposite direction, the mounting sleeves 1.2 are locked against rotation.

Each mounting sleeve 1.2 is adapted to mount one unused or used needle assembly 2 or 2'. Each needle assembly 2, 2' comprises a medical needle 2.1 with an attached needle hub 2.2 for use e.g. with a pen adapter 3.1 of an injection device 3. Each medical needle 2.1 has a distal end for piercing an injection site, e.g. the skin of a user, and a proximal end for piercing a seal of a not shown cartridge containing a medicine to be expelled and which is held in the injection device 3. The needle hub 2.2 is attached to the medical needle 2.1 and is adapted to be connected to the injection device 3.

In the rest position according to figure 1 the cavity 1.5.1 is positioned over the case 1.1 that means the opening 1.4 is closed in such a manner that an entrance to the mounting sleeves 1.2 is closed by the cover element 1.5 and an injection device 3 cannot be inserted.

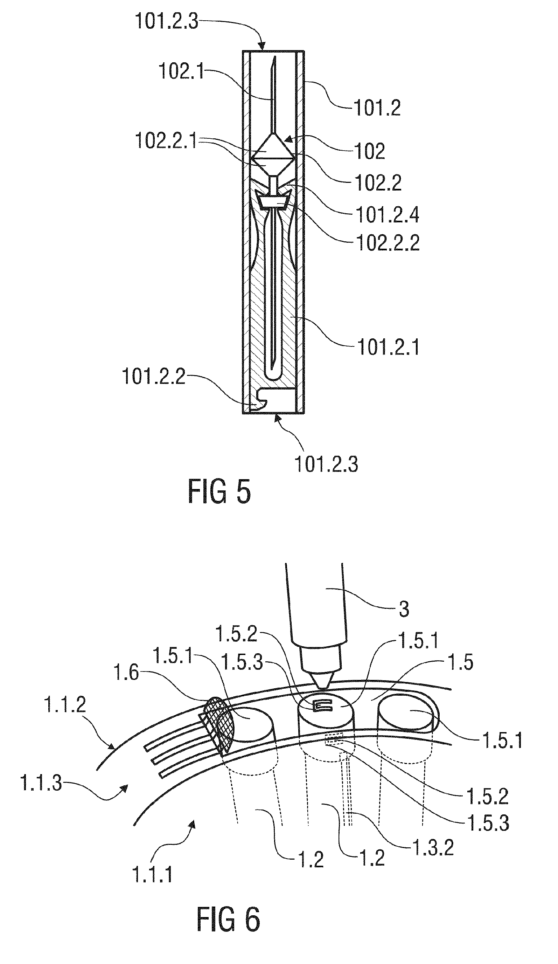

The cover element 1.5 with the cavity 1.5.1 is designed as a sliding window and can be moved from the rest position in figure 1 to the feeding and reinsertion position in figure 2 and further to the disposal position in figure 6 and back to the rest position shown in figure 1 by a sliding assembly 1.6, e.g. a hand lever exemplary shown in figure 6.

In the feeding or reinsertion position according to figure 2, the cavity 1.5.1 is aligned to a mounting sleeve 1.2 and thus to an unused needle assembly 2 mounted in the mounting sleeve 1.2. An injection device 3 can be inserted into the cavity 1.5.1 to withdraw the unused needle assembly 2 from the mounting sleeve 1.2 and fix it by an e.g. snap-fit connection.

After use, the injection device 3 with the attached used needle assembly 2' is put into the cavity 1.5.1 and is moved to the disposal position shown in figure 3 according to arrow P2 in figure 2. Due to this movement, all mounting sleeves 1.2, and thus the inner components of the needle storage magazine 1, are rotated so that an unused needle assembly 2 is moved into the feeding and dispensing position shown in figure 2.

The cavity 1.5.1 cannot move back to the feeding and dispensing position while the injection device 3 is inserted.

When the injection device 3 is extracted from the needle storage magazine 1 the used needle assembly 2' is retained in the mounting sleeve 1.2 and the cavity 1.5.1 is moved back from the disposal position shown in figure 6 to the rest position shown in figure 1 by a not shown resilient element, e.g. a spring. That means that the cavity 1.5.1 is automatically snapped back to the rest position so that the needle storage magazine 1 is closed.

For feeding or reinsertion the needle assembly 2, 2' out from or into the mounting sleeve 1.2, the needle storage magazine 1 comprises a feeding and storage mechanism 1.3. The feeding and storage mechanism 1.3 is arranged within the case 1.1 of the needle storage magazine 1. In particular, the feeding and storage mechanism 1.3 is substantially arranged in the centre of the case 1.1 and opposite to the opening 1.4 positioned in the feeding and reinsertion position according to figure 2.

Figure 4 shows an exemplary embodiment of a mounting sleeve 1.2 with a mounted unused needle assembly 2.

In the exemplary embodiment, the mounting sleeve 1.2 has a cylindrical shape and is closed on both ends by a seal 1.2.3.

The needle hub 2.2 attached to the needle 2.1 has a profile shape, e.g. a circumferential collar 2.2.1, which is adapted to be connected to the injection device 3 e.g. by a snap-fit connection. Alternatively, the needle hub 2.2 can be adapted for other connection types such as for bayonet or thread connection.

A needle support 1.2.1 is arranged within the mounting sleeve 1.2. The needle support 1.2.1 has a profile shape to support the needle assembly 2 in an upright position within the mounting sleeve 1.2. In an exemplary embodiment, the needle support 1.2.1 has an L-profile. One end of the needle support 1.2.1, in particular the end that is directed inwards, is bevelled such that a latching element 1.2.2 is provided. The other end of the needle support 1.2.1 engages the circumferential collar 2.2.1 of the needle assembly 2.

The needle support 1.2.1 is movably arranged within the mounting sleeve 1.2 with respect to the mounting sleeve 1.2. In an exemplary embodiment, the needle support 1.2.1 is movable together with the unused attached needle assembly 2 relative to the mounting sleeve 1.2 to be moved out the unused needle assembly 2 from the mounting sleeve 1.2 for attaching it to the injection device 3 as well as to be moved back and stored the used needle assembly 2' into the mounting sleeve 1.2.

Figure 5 shows an alternative embodiment of a mounting sleeve 101.2 with an attached alternative needle support 101.2.1.

The needle support 101.2.1 has a U-profile to support a needle assembly 102 in an upright position whereby the closed end of the U-profile directed inwards comprises a bent hook to provide a latching element 101.2.2. The opposite open end of the U-profile comprises retaining clips 101.2.4 engaging the needle hub 102.2.

In an exemplary embodiment, the mounting sleeve 101.2 has a cylindrical shape and is closed on both ends by a seal 101.2.3.

The needle hub 102.2 attached to the needle 102.1 has a profile shape, e.g. separate shells 102.2.1, which are adapted to be connected to the injection device 3 e.g. by an alternative snap-fit connection. Furthermore, the needle hub 102.2 comprises a flange 102.2.2 on which the needle support 101.2.1 is detachably connected, e.g. by the retaining clips 101.2.4.

The needle support 101.2.1 with the attached needle assembly 2 is also movably arranged within the mounting sleeve 101.2 with respect to the mounting sleeve 101.2 to be moved out the unused needle assembly 2 from the mounting sleeve 1.2 for attaching it to the injection device 3 as well as to be moved back and stored the used needle assembly 2' into the mounting sleeve 1.2.

Figure 6 shows an enlarged section of an exemplary embodiment of a needle storage magazine 1 with an uncovered opening 1.4 and a cover cavity 1.5.1, which is positioned in the feeding or reinsertion position, especially in the middle position. The cavity 1.5.1 of the cover element 1.5 is designed as a sliding window or mouth which may close or open the case opening 1.4. The cavity 1.5.1 has a substantially cylindrical form and comprises ratchets 1.5.2 formed into an aperture 1.5.3 of the wall.

The sliding assembly 1.6, sliding the cavity 1.5.1, is designed as a hand lever. The sliding assembly 1.6 slides the cavity 1.5.1 from the rest position according to figure 1 (= left position in figure 6) into the feeding or reinsertion position according to figure 2 (= middle position in figure 6). After use, the user inserts the injection device 3 into the cavity 1.5.1 in the middle position (= reinsertion position) and moves it sideways, like a hand gear, to the disposal position (= right position in figure 6). Alternatively, an operating element may be provided to move the opening into the disposal position.

In figure 6 the other positions of the cavity 1.5.1, e.g. the rest position and the disposal position, laterally next to the feeding or reinsertion position, are shown in dotted line.

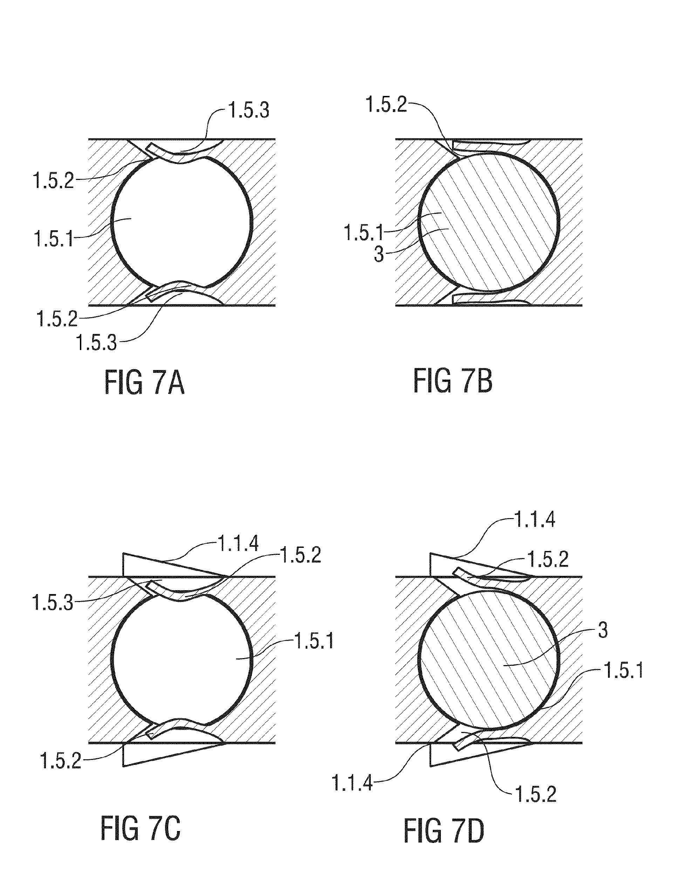

In the disposal position right to the feeding or reinsertion position, the ratchets 1.5.2 snap into corresponding notches 1.1.4 (shown in figures 7C, 7D) formed into the case 1.1 so that the cavity 1.5.1 is locked against a movement back to the feeding or reinsertion position. When the injection device 3 is withdrawn from the needle storage magazine 1 in the disposal position the used needle assembly 2', 102' is retained in the mounting sleeve 1.2, 101.2 by irreversibly closing the retaining clips 101.2.4 on the needle hub 2.2', 102.2'. Further, the ratchets 1.5.2 are unlocked so that the cavity 1.5.1 snaps back to the rest position according to figure 1, the position left to the feeding or reinsertion position, by operating of a not shown resilient element, e.g. a spring.

Figure 7 shows four enlarged sections of the cavity 1.5.1 in different positions.

Figures 7A and 7B show the cavity 1.5.1 in the feeding or reinsertion position, wherein the cavity 1.5.1 is empty and the ratchets 1.5.2 protrude inwards the cavity 1.5.1 shown in figure 7A. Figure 7B shows an injection device 3 inserted into the cavity 1.5.1 so that the ratchets 1.4.1 are bent outwards and engage the inner wall of the case 1.1.

Figures 7C and 7D show the cavity 1.5.1 in the disposal position, wherein the cavity 1.5.1 is empty, the ratchets 1.5.2 protrude inwards the cavity 1.5.1 and do not engage into the notches 1.1.4 shown in figure 7C. Figure 7D shows an injection device 3 inserted into the cavity 1.5.1 so that the ratchets 1.5.2 are bent outwards and engage into the notches 1.1.4 in the inner wall of the case 1.1 to lock the cavity 1.5.1 in the disposal position as long as the injection device 3 is inserted.

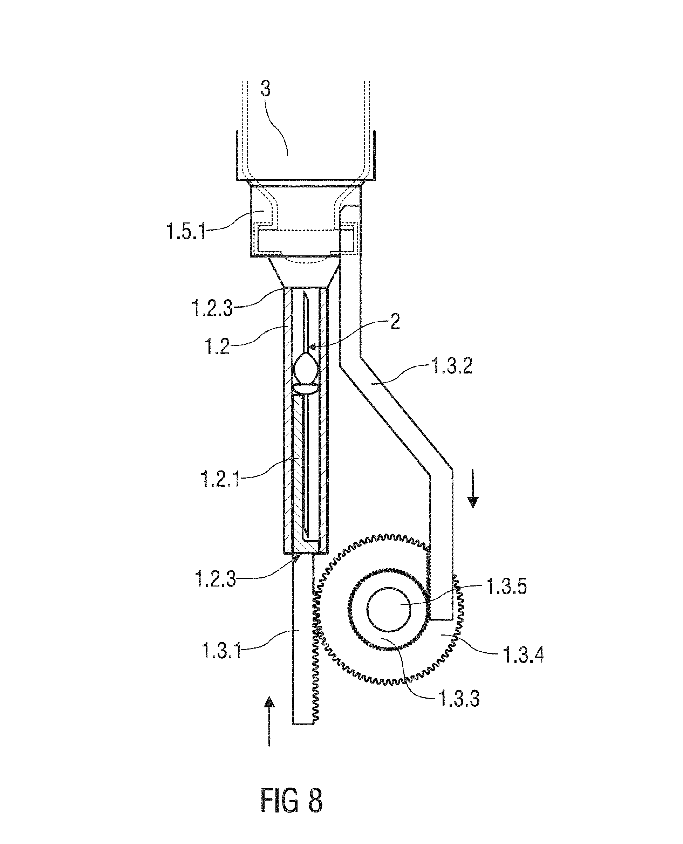

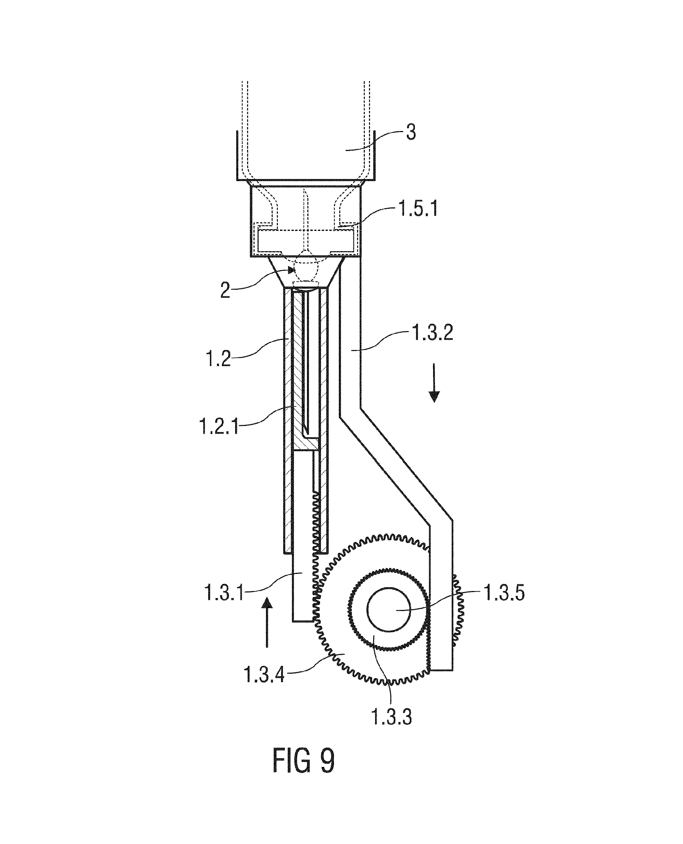

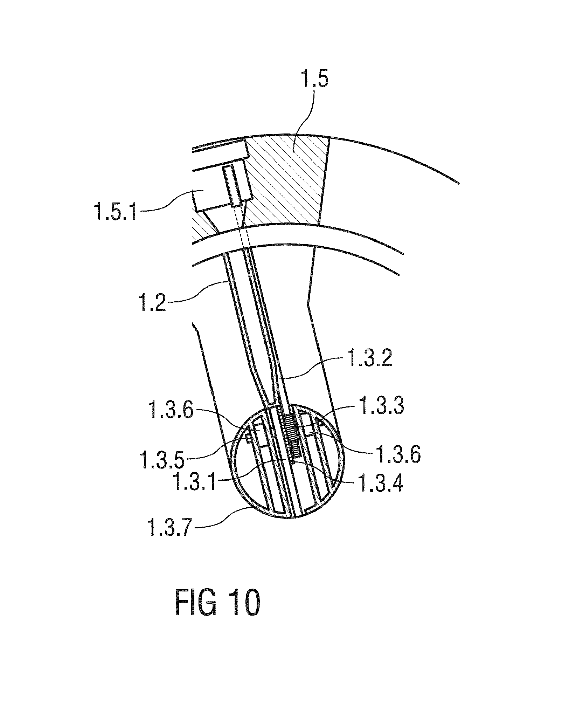

Figures 8 to 10 show sectional views of an exemplary embodiment of a feeding and storage mechanism 1.3 in different positions.

The feeding and storage mechanism 1.3 feeds one of the unused needle assemblies 2 within the mounting sleeve 1.2 to the opening 1.4 which is aligned with the cavity 1.5.1 in the feeding or reinsertion position as well as draws one of the used needle assemblies 2' back into the mounting sleeve 1.2.

In an exemplary embodiment, the feeding and storage mechanism 1.3 is designed as a gear drive. For instance, the gear drive is mounted in a gear casing 1.3.7. The gear drive comprises at least two gear rods 1.3.1, 1.3.2 and at least two gear wheels 1.3.3, 1.3.4 are movably arranged on a gear drive shaft 1.3.5 with bearings 1.3.6. The gear rods 1.3.1, 1.3.2 comprise gear rims which interact with teeth of the gear wheels 1.3.3, 1.3.4.

When an injection device 3 is inserted into the cavity 1.5.1 in the feeding and reinsertion position to pick up an unused sterile needle assembly 2 from the mounting sleeve 1.2, an end of the injection device 3 directed inwards the needle storage magazine 1 engages an end of one of the gear rods 1.3.2 which protrudes over the mounting sleeve 1.2 into the cavity 1.5.1 and pushes it down. Due to this movement, the gear rod 1.3.2 actuates the gear wheel 1.3.3 which actuates another gear wheel 1.3.4 and thus the other gear rod 1.3.1 in such a manner that this gear rod 1.3.1 breaks through the lower seal 1.2.3 of the mounting sleeve 1.2. Thereby, the gear rod 1.3.1 engages the movable needle support 1.2.1 and moves it towards the opening 1.4 and the cavity 1.5.1. The needle 2.1 breaks through the upper seal 1.2.3 and contacts the injection device 3 to reversibly lock at the injection device 3.

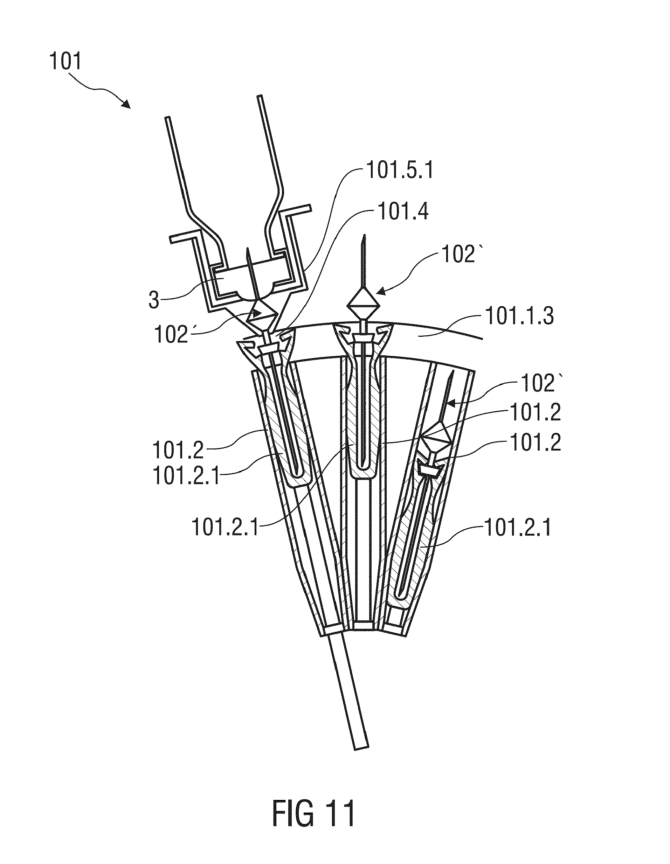

Figures 11 to 13 show in sectional views an exemplary embodiment of a needle storage magazine 101 with used needle assemblies 102' during reinsertion and storing process but without a feeding and storing mechanism for better clarity.

Figure 11 exemplary shows used needle assemblies 102' in three positions within the needle storage magazine 101: during reinsertion into the mounting sleeve 101.2, in an intermediate position as well as in the disposal position.

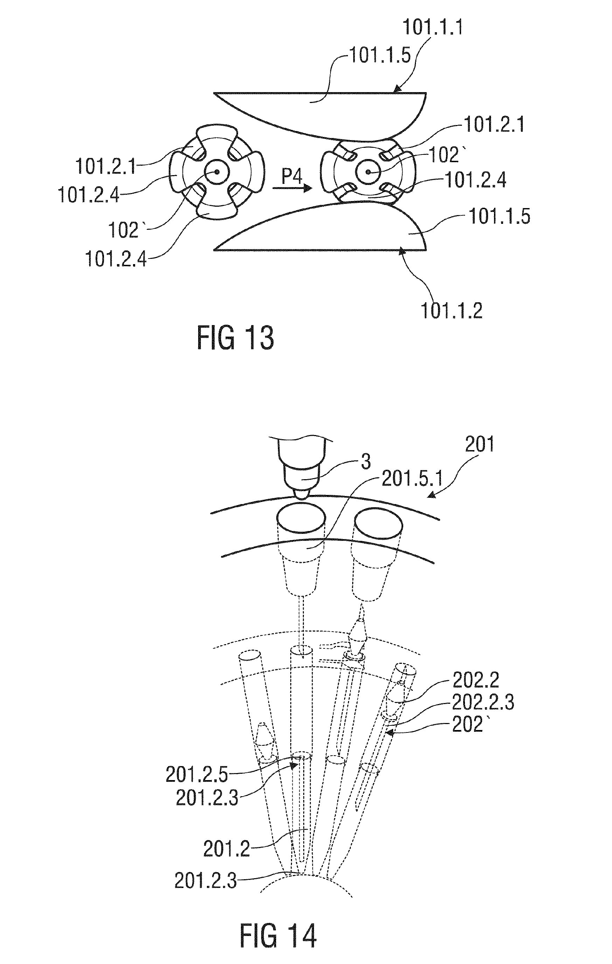

After use, the injection device 3 is put into the cavity 1.5.1 aligned to the opening 1.4 and positioned in the reinsertion position (= left mounting sleeve 101.2 according to figure 11). Subsequently, the injection device 3 mounting into the cavity 1.5.1 is moved sideways to the disposal position according to figure 3 but the used needle assembly 102' is in an intermediate position in which the used needle assembly 102' is half inserted and is fixed in the needle storage magazine 101 by closing retaining elements, e.g. retaining clips 101.2.4 (= middle mounting sleeve 101.2 according to figure 11). The closing process is exemplary shown in figure 13.

To close the retaining clips 101.2.4, the case 101.1 may comprise as a closing mechanism two rips 101.1.5 protruding from the surfaces 101.1.1, 101.1.2 inwards. Due to the movement of the needle support 101.2.1 with the attached used needle assembly 102' according to arrow P4, the retaining clips 101.2.4 of the needle support 101.2.1 are closed by the engaged rips 101.1.5 so that the needle assembly 102' is irreversibly fixed in the needle support 101.2.1.

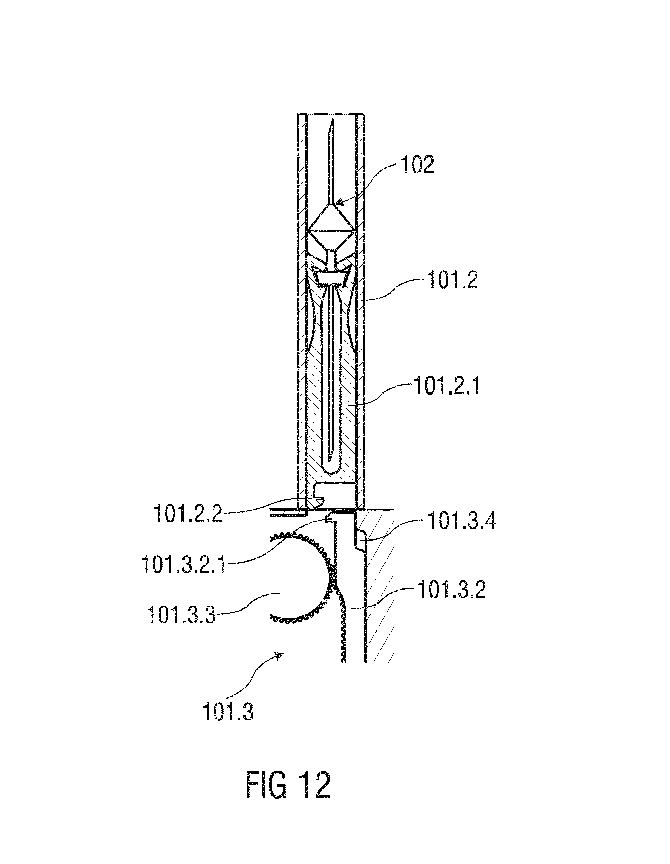

Until the injection device 3 is extracted from the cavity 101.5 in the disposal position, the gear rod 101.3.2 is moved down by a not shown resilient element, e.g. a spring, and pulls the needle support 101.2.1 with the attached and fixed used needle assembly 102' back into the mounting sleeve 101.2 in a disposal position (= right mounting sleeve 101.2 according to figure 11) as shown exemplary in figure 12.

Figure 12 shows in more detail an exemplary embodiment of a detachable connection of the needle support 101.2.1 with a corresponding feeding and storage mechanism 101.3. Figure 12 shows a needle support 101.2.1 according to the exemplary embodiment of figure 5. The needle support 101.2.1 comprises as a latching element 101.2.2 a protruding bent hook. A guiding element 101.3.4, e.g. a guiding surface, moves the gear rod 101.3.2 sideways in such a manner that a latching element 101.3.2.1, formed on the respective end of the gear rod 101.3.2, is engaged on the corresponding latching element 101.2.2 of the needle support 101.2.1 so that the gear rod 101.3.2 is connected to the needle support 101.2.1. Upon removal of the injection device 3 from the cavity 1.5.1 in the disposal position, the gear rod 101.3.2 is moved down driven by a spring and thus the needle support 101.2.1 is moved into the mounting sleeve 101.2 in the disposal position.

Figure 14 shows in a sectional view an exemplary embodiment of a needle storage magazine 201 with a different used needle assembly 202' mountable/mounted in a corresponding mounting sleeve 201.2.

In order to reduce dimensions of the needle storage magazine 201 to fit more needle assemblies 202' into it, the mounting sleeves 201.2 are designed without one of the above described needle supports. Figure 14 shows such a mounting sleeve 201.2 which has a cylindrical shape with thin diameter and comprises a substantially centred passageway 201.2.5 for the needle assembly 202'. The passageway 201.2.5 corresponds with the dimensions and form of the profile of the needle assembly 202'. The needle assembly 202' is held in the mounting sleeve 201.2 by friction.

In an exemplary embodiment, the needle assembly 202' may additionally comprise an elastomer ring 202.2.3 for better friction between the needle assembly 202' and the mounting sleeve 201.2.

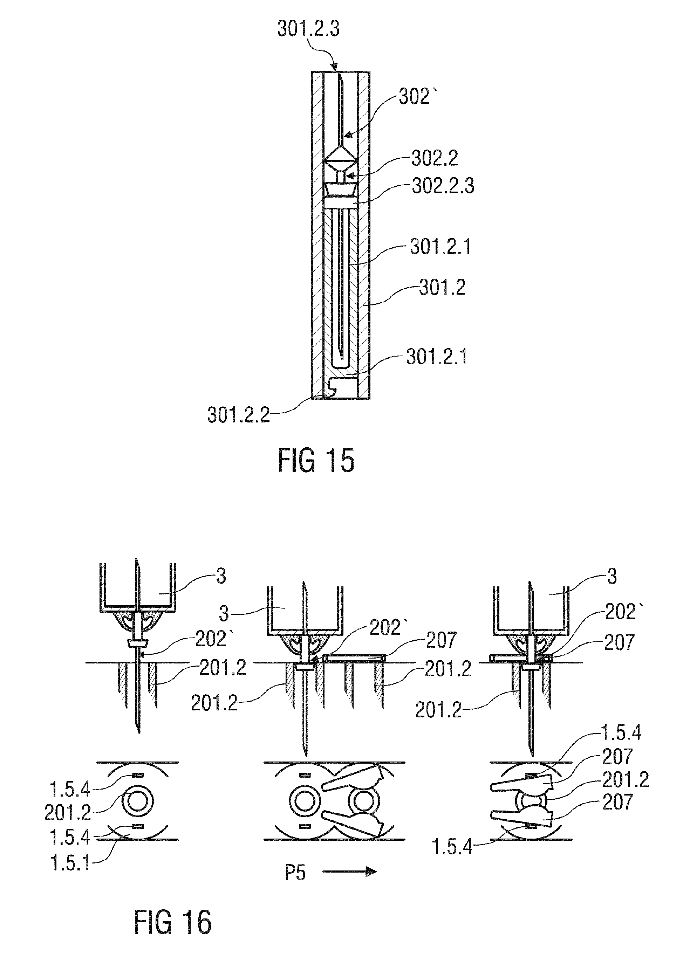

Figure 15 shows a further embodiment of a mounting sleeve 301.2 with a needle support 301.2.1 with an attached needle assembly 302 comprising a friction ring 302.2.3. The friction ring 302.2.3 may be provided as an elastomer ring or rubber ring and is arranged to retain the needle assembly 302, especially the needle hub 302.2 by frictional contact.

Figure 16 shows in a sequence of sectional views and corresponding top views an exemplary embodiment of a needle storage magazine 201 according to figure 14 with used needle assemblies 202' mountable/mounted in mounting sleeves 201.2 in different positions.

For unlinking the used needle assemblies 202' without a needle support with retaining clips (so called slim-needle-tube) according to figure 14 from the injection device 3 and keeping it inside the mounting sleeve 201.2, the reinsertion mechanism is differently designed.

Needle retaining clamps 207 are additionally provided which are pushed together by two pins 1.5.4 fixed on the slidable cover element 1.5, in particular the cavity 1.5.1, when the injection device 3 is moved from the feeding and reinsertion position to the disposal position according to figure 5.

In an exemplary embodiment, the needle retaining clamps 207 are fixed on the inner wall of surface 201.1.1. In particular, a hinge can be added on the elongate side of each clamp 207 (not shown). Furthermore, the hinge may be designed as a prestressed, e.g. spring-loaded connection and is mounted on the above respective pin 1.5.4.

In an alternative embodiment, the pins 1.5.4 of the sliding cover element 1.5 may be avoided if a spring mechanism is provided to push the needle retaining clamps 207 together.

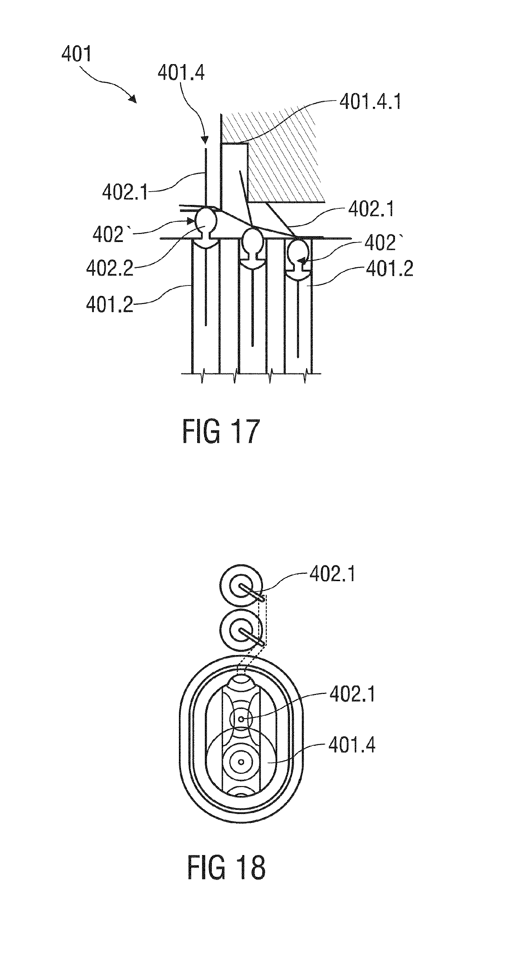

Figure 17 shows another sequence of sectional views of a further exemplary embodiment of a needle storage magazine 401 with used needle assemblies 402' mountable/mounted in mounting sleeves 401.2 in different positions. Instead of fully removing back the needle assemblies 402' into the mounting sleeve 401.2, the top end of the needles 402.1 is bended by a respective inner surface contour 401.4.1 of the opening 401.4.

When the needle storage magazine 401 rotates, the top ends of the needles 402.1 brush along the surface contour 401.4.1 in such a manner that the needle ends are bent so that they do not hinder the injection device 3 to enter the neighboring cavity. As shown in the figures 18 to 19, the needles 402.1 bend tangential to the circle of the mounting sleeves 401.2 and to the side in orthogonal direction. Because the needles 402.1 only bend when the needle storage magazine 401, in particular the mounting sleeves 401.2, are rotated, it is necessary to forward several needles 402.1, instead of only one needle, when the inner components, e.g. the mounting sleeves 401.2, are rotated, to assure that always a bend needle 402.1 is next to the needle under the extraction opening 401.4.

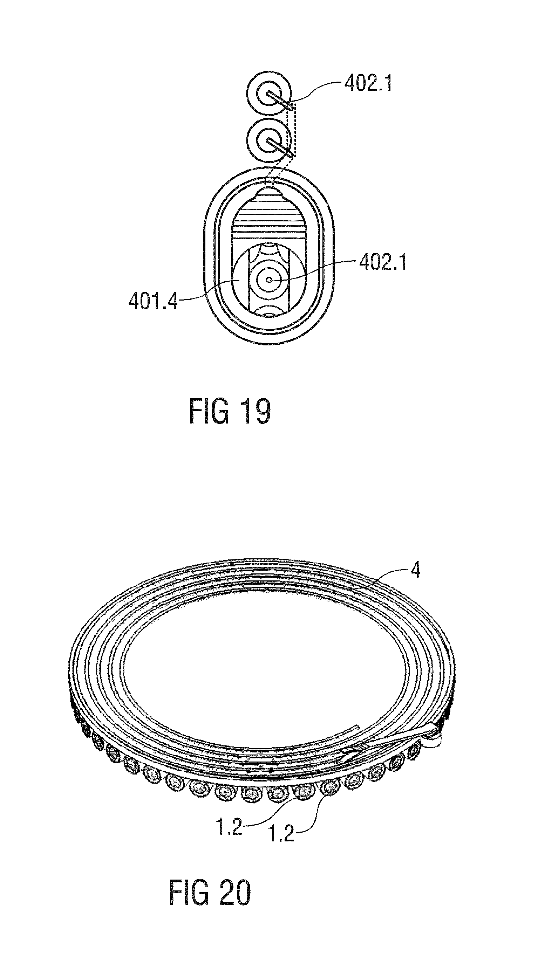

To realize this, the needle storage magazine 401 can be equipped with an uneven number of injection needles and an even number of needles is transformed every needle transporting action. To control this, a ratchet disk 4, as shown in figure 20, can be used.

The described embodiments according to the invention will be used as a needle storage magazine for easy storage and transport of injection pen needles (e.g. for dispensing insulin) and has the following advantages: The invention enables to take out needles from the needle storage magazine and to put used needles back into the needle storage magazine easily and safely. A reuse-prevention prevents the access to used needles. A feeding and storing mechanism mounts a new needle or demounts a used needle automatically to or from the injection device, e.g. an insulin pen.

The use of single needles has the advantage that a considerably higher packing density can be achieved than by the use of common insulin needles, e.g. separately packaged pen needles. So the patient can take along comfortably a needle ration for various days or weeks. The high packing density and the radial arrangement of the needle assemblies gives the needle storage magazine a handy shape that allows an easy pocket transport. The automatic feeding and storing mechanism provides an easy handling and protects the user from needle stick injuries when demounting the needle and from malfunction because of wrong mounting of the needle. By using a perforated tape to seal the mounting sleeves the user does no longer need to remove the sterile barrier manually. This simplifies the steps a user has to take to get a sterile needle from a needle storage magazine.

Furthermore, the integrated reuse prevention protects the user from unintentional reuse of a needle. It also provides a safe disposal of used needles and prevents infections and injuries because of neglectfully stored needles. As the used needles and also the sterile barrier are stored inside the case, there is no waste during the use of the needle storage magazine. If the needle storage magazine will be disposed, the used needles are protected, so there is no injury risk for people who handle the waste.

Further, the use of the injection device as a hand gear for needle transport ensures low operating forces, easy handling and good styling.

## Claims

1. Needle storage magazine (1, 101 to 401) for storing and dispensing a plurality of needle assemblies (2, 102 to 402, 2', 202' to 402'), the needle storage magazine (1, 101 to 401) comprising:
- a plurality of mounting sleeves (1.2, 101.2 to 401.2) each adapted to mount an unused needle assembly (2, 102 to 402) or a used needle assembly (2', 102' to 402'),
- a case (1.1, 101.1 to 401.1) holding the mounting sleeves (1.2, 101.2 to 401.2) side by side, and
- a feeding and storage mechanism (1.3, 101.3 to 401.3) which feeds one of the unused needle assemblies (2, 102 to 402) within the mounting sleeve (1.2, 101.2 to 401.2) to an opening (1.4, 101.4 to 401.4) as well as which draws back one of the used needle assemblies (2', 102' to 402') into the mounting sleeve (1.2, 101.2 to 401.2).

2. Needle storage magazine (1, 101 to 401) according to claim 1,
**characterized in that** the feeding and storage mechanism (1.3, 101.3 to 401.3) is designed as a gear drive.

3. Needle storage magazine (1, 101 to 401) according to claim 1 or 2,
**characterized in that** the feeding and storage mechanism (1.3, 101.3 to 401.3) comprises at least two gear rods (1.3.1, 1.3.2, 101.3.1, 101.3.2 to 401.3.1, 401.3.2) and between them at least one gear wheel (1.3.3, 101.3.3 to 401.3.3) is movably arranged.

4. Needle storage magazine (1, 101 to 401) according to claim 3,
**characterized in that** the mounting sleeve (1.2, 101.2 to 401.2) comprises at its end directed to the feeding and storage mechanism (1.3, 101.3 to 401.3) a latching element (1.2.2, 101.2.2 to 401.2.2) and one of the gear rod (1.3.2, 101.3.2 to 401.3.2) comprises at its end directed to the opening (1.4, 101.4 to 401.4) a corresponding latching element (1.3.2.1, 103.2.1 to 401.3.2.1).

5. Needle storage magazine (1, 101 to 401) according to one of the preceding claims,
**characterized in that** the opening (1.4, 101.4 to 401.4) is formed into a case wall, whereby the opening (1.4, 101.4 to 401.4) is coverable by a cover element (1.5, 101.5 to 401.5) comprising a cavity (1.5.1, 101.5.1 to 401.5.1) and which is movable with respect to the case (1.1, 101.1 to 401.1).

6. Needle storage magazine (1, 101 to 401) according to one of the preceding claims,
**characterized in that** the mounting sleeves (1.2, 101.2 to 401.2) are radially arranged and form together a full circle.

7. Needle storage magazine (1, 101 to 401) according to claim 6,
**characterized in that** the mounting sleeves (1.2, 101.2 to 401.2) are rotatably arranged within the case (1.1, 101.1 to 401.1) in at least one direction and are locked into the opposite direction.

8. Needle storage magazine (1, 101 to 401) according to one of the preceding claims,
**characterized in that** the case (1.1, 101.1 to 401.1) comprises a closing mechanism closing a retaining mechanism of the mounting sleeve (1.2, 101.2 to 401.2) for irreversible retaining an used needle assembly (2', 102' to 402') within the mounting sleeve (1.2, 101.2 to 401.2).

9. Needle storage magazine (1, 101 to 401) according to claim 8,
**characterized in that** the retaining mechanism comprises at least one retaining clip (101.2.4) radially arranged on an inner surface of the mounting sleeve (101.2) on the end of the mounting sleeve (101.2) directed to the opening (101.4).

10. Needle storage magazine (1, 101 to 401) according to claim 8 or 9,
**characterized in that** the retaining clip (101.2.4) is closed by the closing mechanism when a cavity (101.5.1) of a cover element (1.5) is moved from a feeding position to a disposal position.

11. Needle storage magazine (1, 101 to 401) according to one of the preceding claims,
**characterized in that** each mounting sleeve (1.2 to 101.2, 301.2 to 401.2) comprises a needle support (1.2.1 to 101.2.1, 301.2.1 to 401.2.1) arranged within the mounting sleeve (1.2 to 101.2, 301.2 to 401.2).

12. Needle storage magazine (1, 101 to 401) according to one of the preceding claims,
**characterized in that** the needle support (1.2.1 to 101.2.1, 301.2.1 to 401.2.1) has a profile shape to support the needle assembly (2, 2' to 102, 102' and 302, 302' to 402, 402') in an upright position.

13. Needle storage magazine (1) according to one of the preceding claims,
**characterized in that** the needle support (1.2.1) has a L-profile to provide a latching element (1.2.2) by a bent arm directed to a gear drive.

14. Needle storage magazine (101, 301, 401) according to one of the preceding claims,
**characterized in that** the needle support (101.2.1, 301.2.1, 401.2.1) has a U-profile to support the needle assembly (102, 102', 302, 302', 402, 402') in an upright position whereby the end of the U-profile directed to a gear drive comprises a bent latching element (101.2.2, 301.2.2, 401.2.2).

15. Needle storage magazine (101, 301, 401) according to one of the preceding claims,
**characterized in that** the needle assembly (202, 202', 302, 302') comprises a friction ring (202.2.3, 302.2.3).

16. Needle storage magazine (401) according to one of the preceding claims,
**characterized in that** the case (401.1) comprises at least one surface (401.4.1) that protrudes inwards to bend used needle (402.1).

17. Needle storage magazine (101 to 401) according to one of the preceding claims,
**characterized in that** each needle assembly (102, 102' to 402, 402') comprises a needle (102.1 to 402.1) mounted on a needle hub (102.2 to 402.2) wherein the needle hub (102.2 to 402.2) comprises a protruding element on the end which is directed inwards the mounting sleeve (101.2 to 401.2) to be irreversibly lockable at retaining clips (101.2.1 to 401.2.1).

18. Needle storage magazine (1, 101 to 401) according to one of the preceding claims,
**characterized in that** the opening (1.4, 101.4 to 401.4) is coverable by a cover element (1.5, 101.5 to 401.5).

19. Needle storage magazine according to claim 18,
**characterized in that** the cover element (1.5, 101.5 to 401.5) comprises an outer gripping surface and a cavity (1.5.1, 101.5.1 to 401.5.1).
